# EUROPEAN PATENT APPLICATION

(11) **EP 4 253 390 A1**
(43) Date of publication of application: **04.10.2023**
(21) Application number: 21897086.1
(22) Date of filing: 25.11.2021
(51) Int. Cl.: C07F 7/20, C07D 311/74, C07C 39/23, A61K 31/352, A61P 25/04, A61P 29/00

(54) **METHOD FOR PURIFYING CANNABINOID COMPOUNDS**

(30) Priority: 25.11.2020 CN 202011327265
(71) Applicant: Chengdu Baiyu Pharmaceutical Co., Ltd., Chengdu, Sichuan 611130 (CN)
(72) Inventor: SUN, Yi, Chengdu, Sichuan 611130 (CN); XU, Xuezhen, Chengdu, Sichuan 611130 (CN); ZHANG, Jing, Chengdu, Sichuan 611130 (CN); WEI, Yonggang, Chengdu, Sichuan 611130 (CN)
(74) Representative: Kador & Partner Part mbB
(86) International application number: PCT/CN2021/133191
(87) International publication number: WO 2022/111586

(57) **Abstract**

Disclosed is a method for purifying cannabinoid compounds that enables cannabinoid compounds to be obtained more economically and with a better purification effect.

## Description

### Technical Field

The present application relates to the field of compound purification, in particular to a method for purifying cannabinoid compounds.

### Background

Cannabinoid compounds have great application value in pharmaceuticals. For example, it has medical effect or potential for Lennox-Gastaut syndrome, tuberous sclerosis-associated epilepsy, autism, post-traumatic stress disorder, inflammatory heart disease, traumatic brain injury, colon cancer and other indications.

Cannabinoids obtained by synthesis and extraction include various impurities, and the cannabinoid compounds are usually separated and purified by chromatography. However, chromatography not only requires the use of a large amount of solvents in industrialization, but also the purification effect is difficult to meet expectations, greatly limiting the scale and efficiency of the separation method. In addition, most cannabinoid compounds are unstable and prone to rearrangement and oxidation at room temperature, which brings difficulties to the separation and storage of the cannabinoid compounds, particularly the most widely used cannabidiol (CBD) and tetrahydrocannabinol (THC).

There is a need for methods that are cost-effective and suitable for large-scale purification of cannabinoid compounds.

### Summary

The object of the present application is to provide a method for purifying cannabinoid compounds. Compared with the existing purification methods, the method has the features of good purification effect and is suitable for industrial production.

One or more embodiments of the present application provide a method for purifying cannabinoid compounds, stereoisomers, deuterated products or salts thereof, comprising: 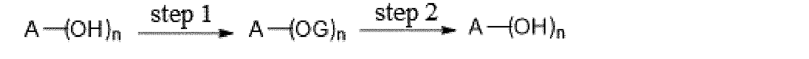 wherein,
A-(OH)ₙ is: 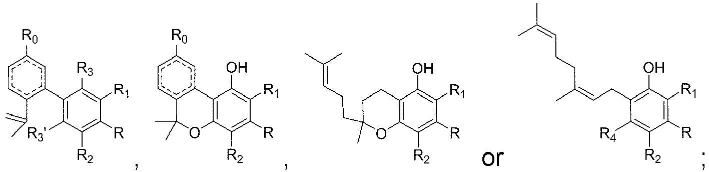
A-(OG)ₙ is: 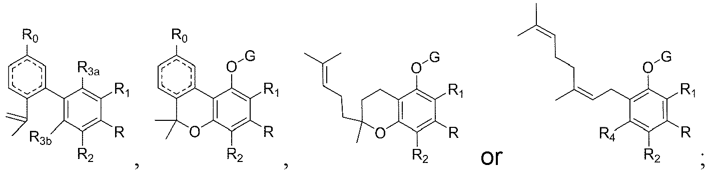 wherein: 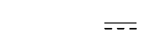
   is a single bond or a double bond;
   R₀ is C₁₋₆ alkyl or C₁₋₆ hydroxyalkyl;
   R is H or C₁₋₁₂ alkyl;
   R₁ is H or -COOH;
   R₂ is H, -COOH or C₁₋₆ alkyl;
   R₃ and R₃' are each independently OH or C₁₋₆ alkoxy, and at least one of R₃ and R₃' is C₁₋₆ alkoxy;
   R₄ is OH, C₁₋₆ alkoxy, -COOH or -OCOC₁₋₆ alkyl;
   G is a silyl protecting group;
   R₃ₐ and R_{3b} are each independently -OG or C₁₋₆ alkoxy, and at least one of R₃ₐ and R_{3b} is -OG;
   n is 1 or 2.

In one or more embodiments, triethylchlorosilane is added in step 1.

In one or more embodiments, tetra-n-butylammonium fluoride is added in step 2.

In one or more embodiments,
A-(OH)ₙ is: 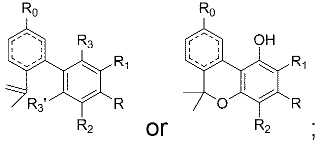
A-(OG)ₙ is: 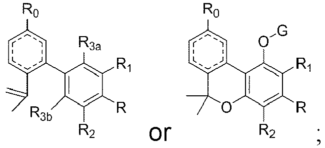 wherein: 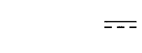
   is a single bond or a double bond;
   R₀ is methyl or -CH₂OH;
   R is H or C₁₋₈ alkyl;
   R₁ is H or -COOH;
   R₂ is H;
   R₃ and R₃' are each independently OH or -OCH₃, and at least one of R₃ and R₃' is OH;
   G is trimethylsilyl, triethylsilyl, triisopropylsilyl, tert-butyldimethylsilyl or tert-butyldiphenylsilyl;
   R₃ₐ and R_{3b} are each independently -OG or -OCH₃, and at least one of R₃ₐ and R_{3b} is -OG;
   n is 1 or 2.

In one or more embodiments, 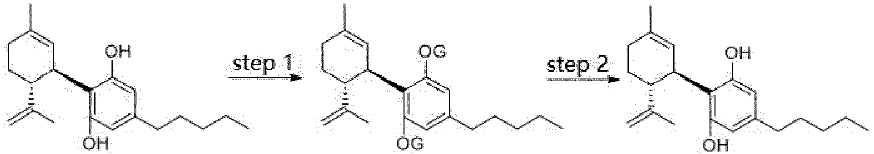 wherein:
G is triethylsilyl or *tert*-butyldimethylsilyl.

In one or more embodiments, the C₁₋₆ alkyl is methyl, and the C₁₋₆ hydroxyalkyl is -CH₂OH.

In one or more embodiments, the C₁₋₁₂ alkyl is pentyl.

In one or more embodiments, the pentyl is n-pentyl.

In one or more embodiments, the C₁₋₆ alkoxy is -OCH₃.

In one or more embodiments, the silyl protecting group is trimethylsilyl, triethylsilyl, triisopropylsilyl, *tert*-butyldimethylsilyl or *tert*-butyldiphenylsilyl.

In one or more embodiments, n is 2.

One or more embodiments of the present application provide compounds having the following structures: 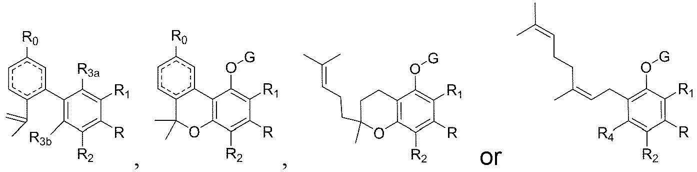 wherein, 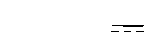
is a single bond or a double bond;
R₀ is C₁₋₆ alkyl or C₁₋₆ hydroxyalkyl;
R is H or C₁₋₁₂ alkyl;
R₁ is H or -COOH;
R₂ is H, -COOH or C₁₋₆ alkyl;
R₃ and R₃' are each independently OH or C₁₋₆ alkoxy, and at least one of R₃ and R₃' is C₁₋₆ alkoxy;
R₄ is OH, C₁₋₆ alkoxy, -COOH or -OCOC₁₋₆ alkyl;
G is a silyl protecting group;
R₃ₐ and R_{3b} are each independently -OG or C₁₋₆ alkoxy, and at least one of R₃ₐ and R_{3b} is -OG.

In one or more embodiments, the C₁₋₆ alkyl is methyl, the C₁₋₆ hydroxyalkyl is -CH₂OH, the C₁₋₁₂ alkyl is pentyl, and the C₁₋₆ alkoxy is -OCH₃, the silyl protecting group is trimethylsilyl, triethylsilyl, triisopropylsilyl, *tert*-butyldimethylsilyl or *tert*-butyldiphenylsilyl.

In one or more embodiments: 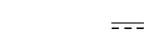
is a single bond or a double bond;
R₀ is methyl or -CH₂OH;
R is H or C₁₋₈ alkyl;
R₁ is H or -COOH;
R₂ is H;
R₃ and R₃' are each independently OH or -OCH₃, and at least one of R₃ and R₃' is OH;
G is trimethylsilyl, triethylsilyl, triisopropylsilyl, *tert*-butyldimethylsilyl or *tert*-butyldiphenylsilyl;
R₃ₐ and R_{3b} are each independently -OG or -OCH₃, and at least one of R₃ₐ and R_{3b} is -OG.

Unless stated to the contrary, the terms used in the specification and claims have the following meanings.

The carbon, hydrogen or oxygen involved in the groups and compounds described in present application all include their isotopes, and which are optionally further replaced by one or more of their corresponding isotopes, wherein the isotopes of carbon include ¹²C, ¹³C and ¹⁴C, and the isotopes of hydrogen include protium (H), deuterium (D, also called heavy hydrogen), tritium (T, also called super-heavy hydrogen), the isotopes of oxygen include ¹⁶O, ¹⁷O and ¹⁸O.

"Alkyl" refers to a linear or branched chain saturated aliphatic hydrocarbon group of 1 to 20 carbon atoms (e.g. 1, 2, 3, 4, 5, 6, 7, 8, 9, 10, 11, 12, 13, 14, 15, 16, 17, 18, 19 or 20 carbon atoms), for example, an alkyl group of 1 to 8 carbon atoms, an alkyl group of 1 to 6 carbon atoms, or an alkyl group of 1 to 4 carbon atoms. Non-limiting examples include methyl, ethyl, n-propyl, isopropyl, n-butyl, sec-butyl, neobutyl, tert-butyl, n-pentyl, isopentyl, neopentyl, n-hexyl and its various branched isomers; when an alkyl group is substituted by a substituent, it may be optionally further substituted by one or more substituents.

"Hydroxyalkyl" refers to an alkyl group substituted with at least one hydroxy group. The alkyl is defined in the same way as for the "alkyl" described above.

"Alkoxy" refers to a group in which at least one carbon atom in an alkyl group is substituted by an oxygen atom. Non-limiting examples include methoxy, ethoxy, *n*-propoxy, isopropoxy, *n*-butoxy, *sec*-butoxy, *tert*-butoxy, *n*-pentoxy, *n*-hexyloxy, cyclopropoxy and cyclobutoxy. The definition of alkyl is the same as the definition of "alkyl" mentioned above.

"Stereoisomer" refers to isomers produced by different arrangements of atoms in a molecule in space, including cis-trans isomers, enantiomers and conformers.

"Optional", "optionally", "selective" or "selectively" means that the subsequently described event or circumstance may, but does not necessarily, occur, and the description includes cases where the event or circumstance occurs and cases where it does not. For example, "heterocyclyl optionally substituted with alkyl" means that the alkyl may, but does not necessarily, be present, and the description includes the case where the heterocyclyl is substituted with alkyl and the case where the heterocyclyl is not substituted with alkyl.

The specification of the present application has described specific embodiments in detail, and those skilled in the art should recognize that the above-mentioned embodiments are exemplary and should not be construed as limitations of the present application. For those skilled in the art, on the premise of not departing from the principles of the present application, some improvements and modifications are made to the present application, and the technical solutions obtained by these improvements and modifications also fall within the protection scope of the claims of the present application.

### Detailed description

The following examples illustrate the technical schemes of the present application in detail, but the protection scope of the present application includes but is not limited thereto.

### Example 1

### (1'R,2'R)-5'-methyl-4-pentyl-2'-(prop-1-en-2-yl)-1',2',3',4'-tetrahydro-[1,1'-biphe nyl]-2,6-diol (Compound 1)

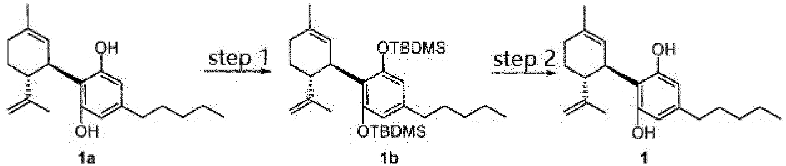

### Step 1:

### (((1'R,2'R)-5'-methyl-4-pentyl-2'-(prop-1-en-2-yl)-1',2',3',4'-tetrahydro-[1,1' -biphenyl]-2,6-diyl)bis(oxy))bis(tert-butyldimethylsilane) (1b)

(1'R,2'R)-5'-methyl-4-pentyl-2'-(prop-1-en-2-yl)-1',2',3',4'-tetrahydro-[1,1'-b iphenyl]-2,6-diol **1a** mixed with impurities **(crude product of Compound 1)** (0.1 g) and imidazole (87mg, 1.28mmol) were dissolved in dichloromethane (0.7ml), and tert-butyldimethylsilyl chloride (TBDMSCI) (116mg, 0.76mmol) was added at 0 °C, which was reacted at 40°C for 3 hours. TLC detected that the reaction was completed, and then the reaction was stopped. The solvent was removed by rotary evaporation, and separated and purified by silica gel column chromatography (dichloromethane/*n*-hexane (v/v)=1/19) to obtain the title compound (((1'R,2'R)-5'-methyl-4-pentyl-2'-(prop-1-en-2-yl)-1',2',3',4'-tetrahydro-[1,1 '-biph enyl]-2,6-diyl)bis(oxy))bis(*tert*-butyldimethylsilane) **1b** (146mg, 84% yield, pale yellow oil).

¹H NMR (300 MHz, Chloroform-*d*) δ 6.19 (s, 2H), 5.22 (t, 1H), 4.45-4.52 (m, 2H), 3.89-3.94 (m, 1H), 3.02 (td, 1H), 2.39-2.44 (m, 2H), 1.94-2.14 (m, 2H), 1.66-1.78 (m, 2H),1.44-1.61 (m, 8H), 1.24-1.34 (m, 6H), 0.84-1.05 (s, 21H), 0.15-0.23 (m, 12H).

LC-MS m/z(ESI)=543.45 [M+1].

### Step 2:

### (1'R,2'R)-5'-methyl-4-pentyl-2'-(prop-l-en-2-yl)-1',2',3',4'-tetrahydro-[1,1'-bi phenyl]-2,6-diol (Compound 1)

(((1'R,2'R)-5'-methyl-4-pentyl-2'-(prop-1-en-2-yl)-1',2',3',4'-tetrahydro-[1,1' -biphenyl]-2,6-diyl)bis(oxy))bis(*tert*-butyldimethylsilane) **1b** (146mg, 0.27mmol) was dissolved in dichloromethane (1ml), tetrahydrofuran solution of tetra-n-butylammonium fluoride (0.54ml, 0.54mmol) was added at 0 °C, and which was reacted at 0 °C for 30 minutes. TLC detected that the reaction was completed, and then the reaction was stopped. The solvent was removed by rotary evaporation, and separated and purified by silica gel column chromatography (ethyl acetate/*n*-hexane (v/v)=2/8) to obtain the title compound (1'R,2'R)-5'-methyl-4-pentyl-2'-(prop-1-en-2-yl)-1',2',3',4'-tetrahydro-[1,1'-biphe nyl]-2,6-diol **(Compound 1)** (80mg, 95% yield, >99% purity, white solid).

¹H NMR (300 MHz, DMSO-*d*₆) δ 8.61 (s, 2H), 5.99 (s, 2H), 5.06 (s, 1H), 4.38-4.47 (m, 2H), 3.80 (d, 1H), 2.96-3.04 (m, 1H), 2.25-2.30 (m, 2H), 1.86-2.07 (m, 2H), 1.42-1.64 (m, 10H), 1.20-1.28 (m, 4H), 0.83 (t, 3H).

LC-MS m/z(ESI)=315.47 [M+1].

### Example 2

### (1'R,2'R)-5'-methyl-4-pentyl-2'-(prop-1-en-2-yl)-1',2',3',4'-tetrahydro-[1,1'-biphe nyl]-2,6-diol (Compound 1)

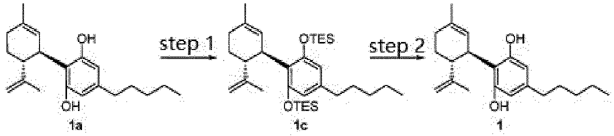

### Step 1

### (((1'R,2'R)-5'-methyl-4-pentyl-2'-(prop-1-en-2-yl)-1',2',3',4'-tetrahydro-[1,1' -biphenyl]-2,6-diyl)bis(oxy))bis(triethylsilane) (1c)

(1'R,2'R)-5'-methyl-4-pentyl-2'-(prop-1-en-2-yl)-1',2',3',4'-tetrahydro-[1,1'-b iphenyl]-2,6-diol **1a** mixed with impurities **(crude product of Compound 1)** (0.26g) and imidazole (0.17g, 2.5mmol) were dissolved in dichloromethane (1.8ml), and triethylchlorosilane (TESCI) (320mg, 2.1mmol) was added at 0 °C, which was reacted at 40 °C for 3 hours. TLC detected that the reaction was completed, and then the reaction was stopped. The solvent was removed by rotary evaporation, and separated and purified by silica gel column chromatography (dichloromethane/*n*-hexane (v/v)=1/19) to obtain the title compound (((1'R,2'R)-5'-methyl-4-pentyl-2'-(prop-1-en-2-yl)-1',2',3',4'-tetrahydro-[1,1'-biph enyl]-2,6-diyl)bis(oxy))bis(triethylsilane) **1c** (433mg, 99% yield, pale yellow oil).

¹H NMR (300 MHz, Chloroform-*d*) δ 6.16 (s, 2H), 5.19 (s, 1H), 4.45-4.49 (m, 2H), 3.85-3.89 (m, 1H), 2.91-2.99 (m, 1H), 2.42 (t, 2H), 1.94-2.14 (m, 2H), 1.48-1.79 (m, 12H), 1.18-1.35 (m, 4H), 0.69-0.99 (m, 33H).

LC-MS m/z(ESI)=543.45 [M+1].

### Step 2:

### (1'R,2'R)-5'-methyl-4-pentyl-2'-(prop-l-en-2-yl)-1',2',3',4'-tetrahydro-[1,1'-bi phenyl]-2,6-diol (Compound 1)

(((1'R,2'R)-5'-methyl-4-pentyl-2'-(prop-1-en-2-yl)-1',2',3',4'-tetrahydro-[1,1' -biphenyl]-2,6-diyl)bis(oxy))bis(triethylsilane) **(1c)** (433mg, 0.8mmol) was dissolved in anhydrous tetrahydrofuran (4ml), tetrahydrofuran solution of tetra-n-butylammonium fluoride (1.8ml, 1.8mmol) was added at 0 °C, and which was reacted at 0 °C for 30 minutes. TLC detected that the reaction was completed, and then the reaction was stopped. The solvent was removed by rotary evaporation, and separated and purified by silica gel column chromatography (ethyl acetate/*n*-hexane (v/v)=2/8) to obtain the title compound (1'R,2'R)-5'-methyl-4-pentyl-2'-(prop-1-en-2-yl)-1',2',3',4'-tetrahydro-[1,1'-biphe nyl]-2,6-diol **(Compound 1)** (250mg, 99% yield, >99% purity, white solid).

¹H NMR (300 MHz, DMSO-*d*₆) δ 8.61 (s, 2H), 5.99 (s, 2H), 5.06 (s, 1H), 4.38-4.47 (m, 2H), 3.80 (d, 1H), 2.96-3.04 (m, 1H), 2.25-2.30 (m, 2H), 1.86-2.07 (m, 2H), 1.42-1.64 (m, 10H), 1.20-1.28 (m, 4H), 0.83 (t, 3H).

LC-MS m/z(ESI)=315.47 [M+1].

## Claims

1. A method for purifying cannabinoid compounds, stereoisomers, deuterated products or salts thereof, comprising: 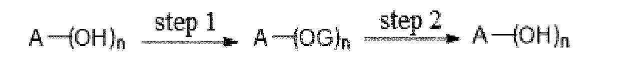 wherein,
A-(OH)ₙ is: 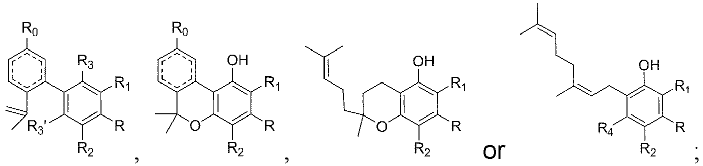
A-(OG)ₙ is: 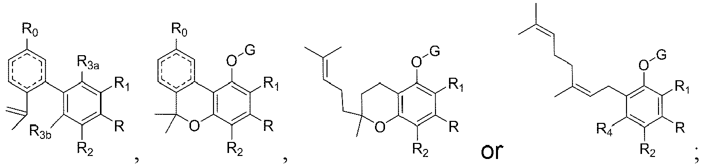 wherein, 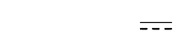
is a single bond or a double bond;
R₀ is C₁₋₆ alkyl or C₁₋₆ hydroxyalkyl;
R is H or C₁₋₁₂ alkyl;
R₁ is H or -COOH;
R₂ is H, -COOH or C₁₋₆ alkyl;
R₃ and R₃*'* are each independently OH or C₁₋₆ alkoxy, and at least one of R₃ and R₃' is C₁₋₆ alkoxy;
R₄ is OH, C₁₋₆ alkoxy, -COOH or -OCOC₁₋₆ alkyl;
G is a silyl protecting group;
R₃ₐ and R_{3b} are each independently -OG or C₁₋₆ alkoxy, and at least one of R₃ₐ and R_{3b} is -OG;
n is 1 or 2.

2. The method for purifying cannabinoid compounds according to claim 1, wherein
A-(OH)ₙ is: 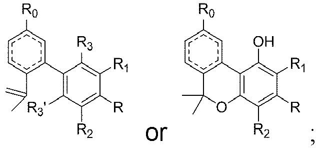
A-(OG)ₙ is: 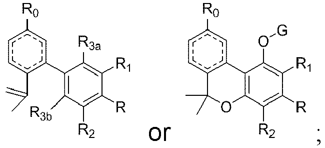 wherein: 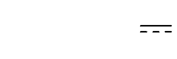
is a single bond or a double bond;
R₀ is methyl or -CH₂OH;
R is H or C₁₋₈ alkyl;
R₁ is H or -COOH;
R₂ is H;
R₃ and R₃' are each independently OH or -OCH₃, and at least one of R₃ and R₃' is OH;
G is trimethylsilyl, triethylsilyl, triisopropylsilyl, *tert*-butyldimethylsilyl or *tert*-butyldiphenylsilyl;
R₃ₐ and R_{3b} are each independently -OG or -OCH₃, and at least one of R₃ₐ and R_{3b} is -OG;
n is 1 or 2.

3. The method for purifying cannabinoid compounds according to claim 1 or 2, wherein 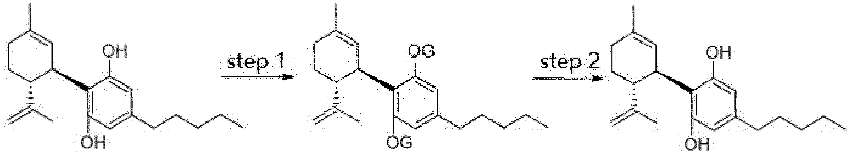 wherein:
G is triethylsilyl or *tert*-butyldimethylsilyl.

4. The method for purifying cannabinoid compounds according to claim 1, wherein the C₁₋₆ alkyl is methyl, and the C₁₋₆ hydroxyalkyl is -CH₂OH.

5. The method for purifying cannabinoid compounds according to claim 1, wherein the C₁₋₁₂ alkyl is pentyl, preferably, the pentyl is *n*-pentyl.

6. The method for purifying cannabinoid compounds according to claim 1, wherein the C₁₋₆ alkoxy is -OCH₃.

7. The method for purifying cannabinoid compounds according to claim 1, wherein the silyl protecting group is trimethylsilyl, triethylsilyl, triisopropylsilyl, *tert*-butyldimethylsilyl or *tert*-butyldiphenylsilyl.

8. The method for purifying cannabinoid compounds according to claim 1, wherein n is 2.

9. A compound having the following structure: 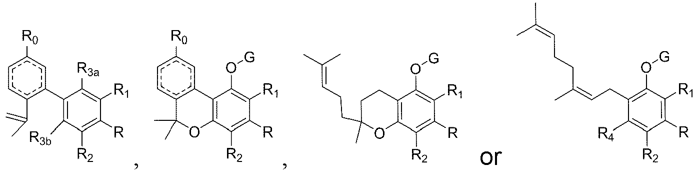 wherein: 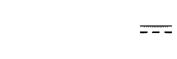
is a single bond or a double bond;
R₀ is C₁₋₆ alkyl or C₁₋₆ hydroxyalkyl;
R is H or C₁₋₁₂ alkyl;
R₁ is H or -COOH;
R₂ is H, -COOH or C₁₋₆ alkyl;
R₃ and R₃' are each independently OH or C₁₋₆ alkoxy, and at least one of R₃ and R₃' is C₁₋₆ alkoxy;
R₄ is OH, C₁₋₆ alkoxy, -COOH or -OCOC₁₋₆ alkyl;
G is a silyl protecting group;
R₃ₐ and R_{3b} are each independently -OG or C₁₋₆ alkoxy, and at least one of R₃ₐ and R_{3b} is -OG;
preferably, the C₁₋₆ alkyl is methyl, the C₁₋₆ hydroxyalkyl is -CH₂OH, the C₁₋₁₂ alkyl is pentyl, and the C₁₋₆ alkoxy is -OCH₃, the silyl protecting group is trimethylsilyl, triethylsilyl, triisopropylsilyl, tert-butyldimethylsilyl or tert-butyldiphenylsilyl.

10. The compound according to claim 9, wherein 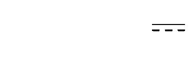
is a single bond or a double bond;
R₀ is methyl or -CH₂OH;
R is H or C₁₋₈ alkyl;
R₁ is H or -COOH;
R₂ is H;
R₃ and R₃' are each independently OH or -OCH₃, and at least one of R₃ and R₃' is OH;
G is trimethylsilyl, triethylsilyl, triisopropylsilyl, tert-butyldimethylsilyl or tert-butyldiphenylsilyl;
R₃ₐ and R_{3b} are each independently -OG or -OCH₃, and at least one of R₃ₐ and R_{3b} is -OG.
